# EUROPEAN PATENT APPLICATION

(11) **EP 4 338 637 A1**
(43) Date of publication of application: **20.03.2024**
(21) Application number: 22916260.7
(22) Date of filing: 25.07.2022
(51) Int. Cl.: A44C 5/00, A44C 5/24, H05F 3/02

(54) **ANKLET FOR EARTH GROUNDING AND ELECTROSTATIC RELEASE**

(30) Priority: 28.12.2021 KR 20210189103
(71) Applicant: Jang, Yoon Seop, Jeollanam-do 57018 (KR)
(72) Inventor: Jang, Yoon Seop, Jeollanam-do 57018 (KR)
(74) Representative: M. Zardi & Co S.A.
(86) International application number: PCT/KR2022/010891
(87) International publication number: WO 2023/128105

(57) **Abstract**

The present invention is intended to provide an anklet for earth grounding and electrostatic release, the anklet including: an anklet part made of a material capable of conducting electricity, and configured to be worn to come into close contact with an ankle; a connector connected to one side of the anklet part; and a ground part connected to the connector, electrically connected to the anklet part, and grounded to a surface an earth. The effect of the present invention is to provide the anklet that can charge the earth's energy through earthing and can also rapidly and completely discharge static electricity generated inside and outside the human body, thereby enabling the health of the human body to be maintained, which is a useful invention.

## Description

### Technical Field

The present invention relates generally to an anklet for earth grounding and electrostatic release, and more specifically to an anklet for maintaining health by charging the earth's energy through earthing and discharging (grounding) static electricity, generated inside and outside the human body, through the surface of the earth.

### Background Art

Earthing refer to maintaining health by electrically connecting the human body through contact with the earth, i.e., the ground (the ground surface), and charging the human body with the earth's energy. Recently, earthing is a type of alternative medicine that is used to prevent diseases and also treat inflammation and pain.

The pioneer of earthing is Clinton Ober of the United States. It began with the question of whether humans are unable to maintain their health by being cut off from the natural electric charge of the earth's surface due to anklets with soles made of materials such as plastic or rubber for a long time. Since then, it has been proven as a fact through scientific research by various researchers, including many biophysicists, electrophysiologists, doctors, and exercise physiologists.

As for the effects of earthing, the world is paying attention to the effects of 'earthing' on the human body not only overseas but also domestically to the extent that the Korean Society of Analytical Science presented a presentation on the topic of 'Effects of electromagnetic waves on the human body and earthing effects' at the 53rd fall conference.

The phenomenon in which an object that gains an electron when the electron moves takes on a negative charge, and an object that loses an electron takes on a positive charge is called electrification. Furthermore, static electricity is a phenomenon caused by such electrification. Such static electricity occurs when two or more insulators rub against each other. Even within the human body, as blood circulates throughout the body at high speed, static electricity is generated due to friction between red blood cells and blood vessels and the friction between red blood cells and red blood cells.

Furthermore, static electricity is generated in the tissues of the circulatory system such as blood vessels, lymph vessels, red blood cells, and lymph in which there are blood and lymph flows in the human body. Static electricity is also generated in the tissues of the digestive system where food enters. Static electricity is also generated in the tissues of the lungs and bronchial tubes of the respiratory system, where air flows in and out through breathing.

Static electricity generated inside the human body as described above accumulates in fat cells and cell membranes that have high electrical resistance. Such accumulated static electricity in the human body causes discharge and damages nerve cells. Accordingly, when the static electricity accumulated in the human body is not discharged out of the body, it may cause disease. The static electricity must be expelled from the human body as rapidly and completely as possible.

Grounding is a very important means for dissipating static electricity, charged on an object, to the ground. As for static electricity, grounding is required for a conductor (conductive & dissipative) having a specific resistance of 108 Ω·m or less.

In general, contact with the surface of the earth using a conductor is called grounding. For earth grounding and electrostatic release from the human body, it is desirable to form a grounding means between the human body and the surface of the earth. Conventionally, electricity is conducted between the human body and the surface of the earth by forming a ground conductor on an insole or a shoe. However, this method is limited to application to an insole or a shoe. Therefore, there are disadvantages in that the method cannot be used when the insole or the shoe is changed and this structure needs to be applied to all insoles or shoes that are worn by a user.

### Disclosure

### Technical Problem

An object of the present invention is to charge the earth's energy through earthing and rapidly and completely discharge static electricity, generated inside and outside the human body, out of the human body, thereby overcoming the problems of the conventional grounding means used in an insole or an anklet.

### Technical Solution

In orderto accomplish the above object, an embodiment of the present invention provides an anklet for earth grounding and electrostatic release, the anklet including: an anklet part made of a material capable of conducting electricity, and configured to be worn to come into close contact with an ankle; a connector connected to one side of the anklet part; and a ground part connected to the connector, electrically connected to the anklet part, and grounded to a surface an earth.

According to an embodiment of the present invention, a length adjustment portion having a predetermined length is formed on one side or each of both sides of each of the anklet part and the ground part.

According to an embodiment of the present invention, the length adjustment portion is formed of a chain, and a hook is formed on one side of the length adjustment portion.

According to an embodiment of the present invention, the anklet part and the ground part are each formed by successively connecting chain segments or spherical metals to each other so that they can conduct electricity to each other.

### Advantageous Effects

The effect of the above-described present invention is to provide the anklet that can charge the earth's energy through earthing and can also rapidly and completely discharge static electricity generated inside and outside the human body, thereby enabling the health of the human body to be maintained, which is a useful invention.

### Description of Drawings

FIG. 1 is an exploded perspective view showing one embodiment of the anklet of the present invention;
FIG. 2 is a perspective view of the combined state of FIG. 1;
FIG. 3 is a combined perspective view showing the length-adjusted states of an anklet part and a ground part in the present invention;
FIG. 4 is a reference diagram showing the state of use of the present invention;
FIG. 5 is an exploded perspective view showing another embodiment of the anklet of the present invention;
FIG. 6 is a perspective view of the combined state of FIG. 5; and
FIG. 7 is a combined perspective view showing the length-adjusted states of an anklet part and a ground part in FIG. 6.

### Mode for Invention

Embodiments of the present invention will be described in detail below with reference to the accompanying drawings so that those having ordinary skill in the art to which the present invention pertains can easily practice the present invention. However, the present invention may be implemented in various different forms and is not limited to the embodiments described herein. The terminology used herein is only intended to refer to specific embodiments and is not intended to limit the present invention. Furthermore, singular forms used herein also include plural forms, unless phrases clearly indicate the contrary. The meaning of "including" used herein specifies one or more specific features, regions, integers, steps, operations, elements, and/or component, and does not exclude the presence or addition of another specific feature, region, integer, step, operation, element, component, and/or group. Although not defined differently, all terms including technical and scientific terms used herein have meanings identical to those generally understood by those having ordinary skill in the art to which the present invention pertains. The terms defined in commonly used dictionaries are further interpreted as having meanings consistent with the related technical literature and currently disclosed technology, and are not interpreted as ideal or official meanings unless defined otherwise.

Embodiments of the present invention described with reference to the perspective views specifically represent ideal embodiments of the present invention. As a result, various variations of illustrations, e.g., variations of manufacturing methods and/or specifications, are expected. Accordingly, the embodiments are not limited to the specific shapes of illustrated areas and also include, e.g., variations of shapes attributable to manufacturing. For example, areas shown or described as flat may generally have rough/rough and non-linear characteristics. Furthermore, portions shown as having sharp angles may be rounded. Accordingly, the areas shown in the drawings are only approximate in nature, and their shapes are not intended to illustrate the exact shapes of the areas, nor are they intended to narrow the scope of the present invention.

Preferred embodiments of an anklet for earth grounding and electrostatic release according to the present invention will be described in detail below with reference to the accompanying drawings.

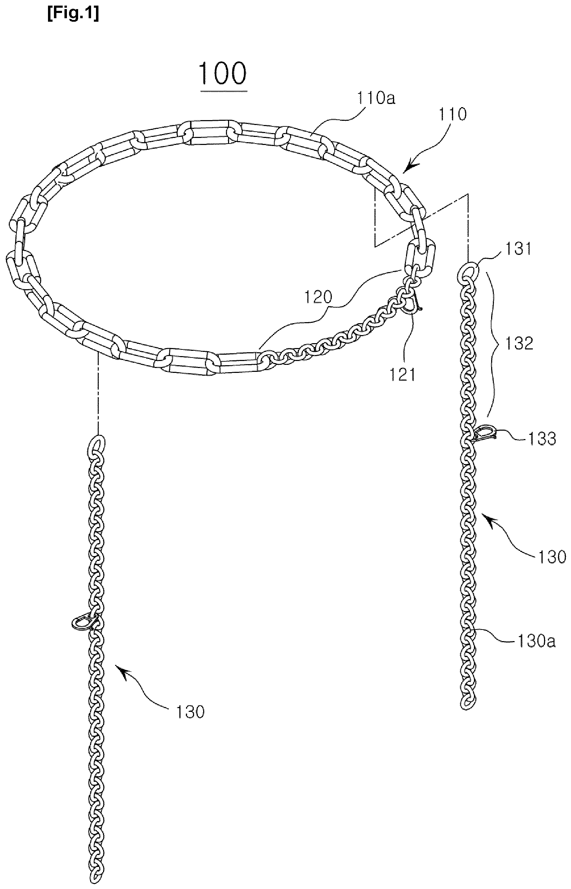

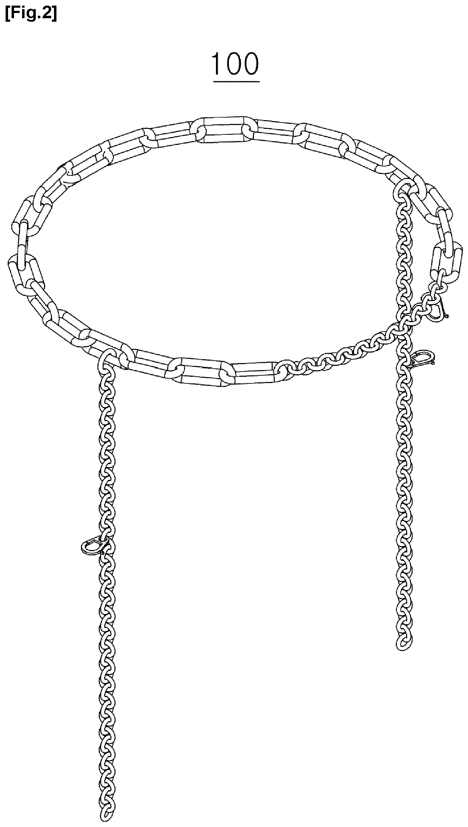

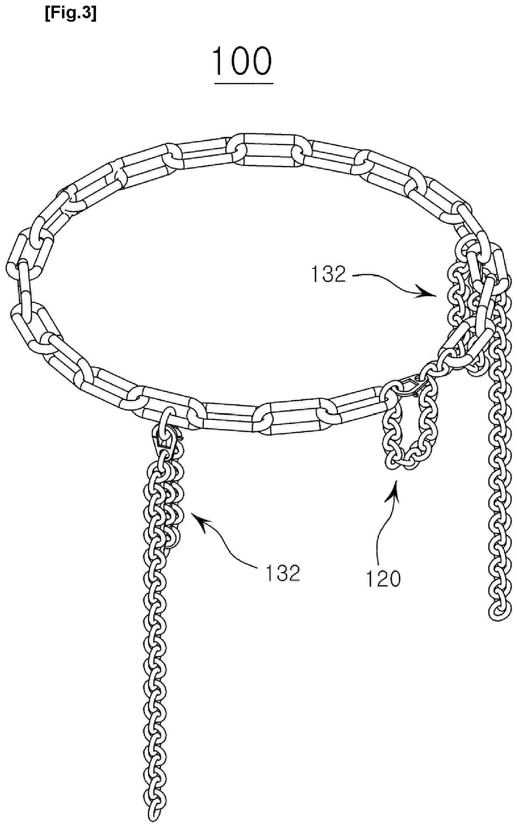

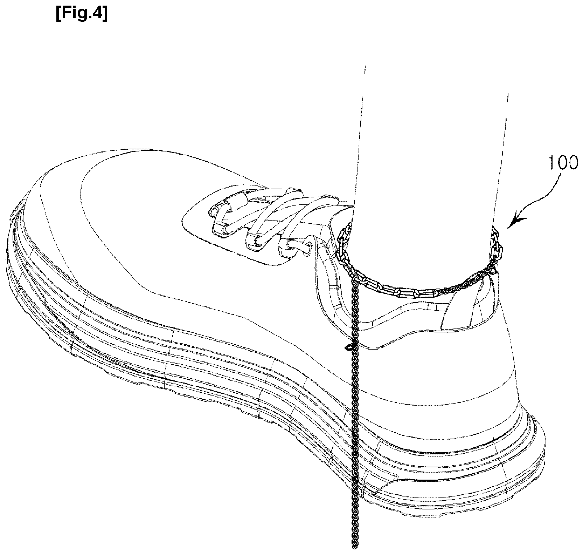

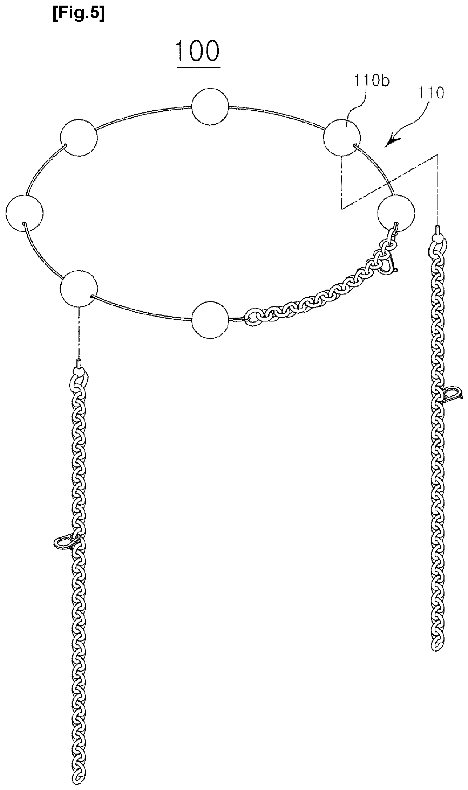

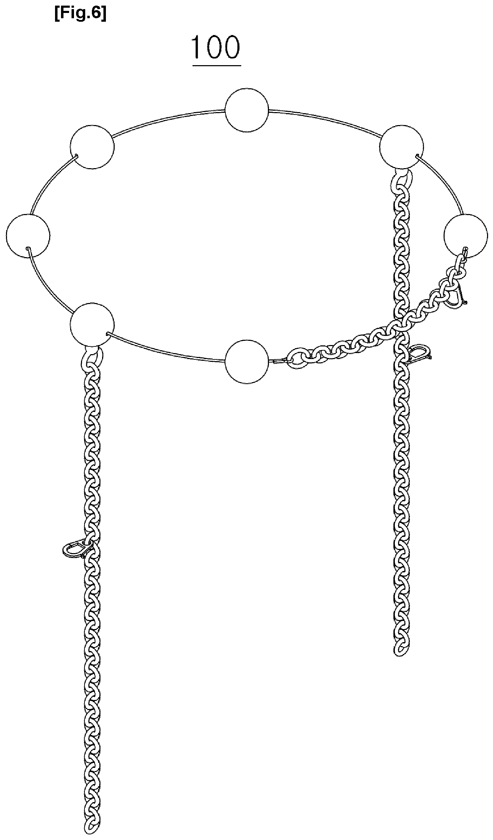

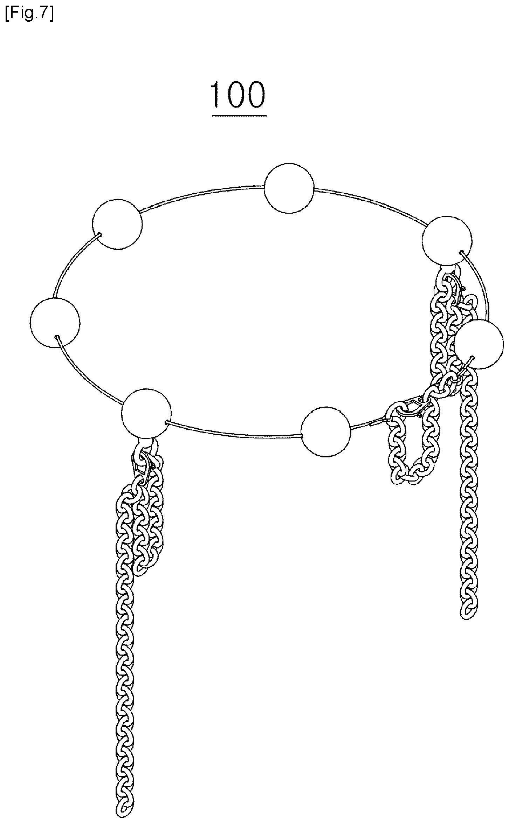

FIG. 1 is an exploded perspective view showing one embodiment of the anklet of the present invention, FIG. 2 is a perspective view of the combined state of FIG. 1, FIG. 3 is a combined perspective view showing the length-adjusted states of an anklet part and a ground part in the present invention, FIG. 4 is a reference diagram showing the state of use of the present invention, FIG. 5 is an exploded perspective view showing another embodiment of the anklet of the present invention, FIG. 6 is a perspective view of the combined state of FIG. 5, and FIG. 7 is a combined perspective view showing the length-adjusted states of an anklet part and a ground part in FIG. 6.

First, it should be noted that in the drawings, identical components or parts are denoted by the same reference numerals whenever possible. Furthermore, in the following description of the present invention, detailed descriptions of related well-known functions or configurations will be omitted in orderto make the gist of the present invention unambiguous.

The anklet 100 of the present invention includes: an anklet part 110 made of a material capable of conducting electricity, and configured to be worn to come into close contact with the ankle; a connector 131 connected to one side of the anklet part 110; and a ground part 130 connected to the connector 131, electrically connected to the anklet part 110, and grounded to the surface the earth.

A length adjustment portion 120 having a predetermined length is formed on one side of the anklet part 110. This is a structure intended to match the diameters of wearers' ankles. The reason for this is that the magnitude of the diameter of a wearer varies from person to person.

In this case, the ground part 130 may be formed on one side of the anklet part 110, or may be formed on each of both sides of the anklet part 110. The ground part 130 is configured to pass by an ankle bone area so that grounding can be facilitated when a wearer wears a shoe.

It is desirable that a length adjustment portion 131 having a predetermined length be formed in the ground part 130. This is a structure intended to match the different lengths of wearers' ankles.

The length adjustment portions 120 and 131 of the anklet part 110 and the ground part 130 are each made of a chain or tether structure. Hooks 121 and 133 are each formed on one side of each of the length adjustment portions 120 and 131, and the length of each of the anklet part 110 and the ground part 130 is adjusted in such a manner that the hook is coupled to one side of the chain or the tether.

The anklet part 110 and the ground part 130 each have a structure in which metal chain or tether rings, spherical metals or precious metals, and/or the like are successively connected to each other such that they can conduct electrically to each other.

In particular, it is preferable that the individual rings 110a and 130a of the chain or tether forming each of the anklet part 110 and the ground part 130 are each formed in an oval or circular shape. The shape and structure of them may be formed in various forms other than the oval or circular form. Above all, they are not limited to a specific shape and structure in the present invention as long as they can conduct electricity.

As described above, the ground part 130 is configured to extend from the anklet part 110 and be grounded to the surface of the earth. In this case, the connector 131 capable of conducting electricity is formed at one end of the ground part 130 and is then connected to the anklet part 110. It is obvious that the connector 131 may be composed of any one of the rings 110a and 130a of the chain or the tether constituting the ground part 130.

While the present invention has been described with reference to the limited embodiments as described above, the present invention is not limited thereto. Various modifications and variations may be made by those having ordinary skill in the art to which the present invention pertains within the technical spirit of the present invention, the scope of the appended claims and the equivalent range thereof.

## Claims

1. An anklet for earth grounding and electrostatic release, the anklet comprising:
an anklet part made of a material capable of conducting electricity, and configured to be worn to come into close contact with an ankle;
a connector connected to one side of the anklet part; and
a ground part connected to the connector, electrically connected to the anklet part, and grounded to a surface an earth.

2. The anklet of claim 1, wherein a length adjustment portion having a predetermined length is formed on one side or each of both sides of each of the anklet part and the ground part.

3. The anklet of claim 1, wherein the length adjustment portion is formed of a chain, and a hook is formed on one side of the length adjustment portion.

4. The anklet of claim 1, wherein the anklet part and the ground part are each formed by successively connecting chain segments or spherical metals to each other so that they can conduct electricity to each other.
